**Europäisches Patentamt**

(19) **European Patent Office**

**Office européen des brevets**

(11) Veröffentlichungsnummer: **0 124 686**
**B1**

(12) # EUROPÄISCHE PATENTSCHRIFT

(45) Veröffentlichungstag der Patentschrift:
26.08.87

(21) Anmeldenummer: **84101262.8**

(22) Anmeldetag: **08.02.84**

(51) Int. Cl.⁴: **A 61 B 17/22**

(54) **Funkenstrecke zur Erzeugung von Stosswellen für die beführungsfreie Zertrümmerung von Konkrementen in Körpern von Lebewesen.**

(30) Priorität: **07.05.83 DE 3316837**

(43) Veröffentlichungstag der Anmeldung:
**14.11.84 Patentblatt 84/46**

(45) Bekanntmachung des Hinweises auf die Patenterteilung:
**26.08.87 Patentblatt 87/35**

(84) Benannte Vertragsstaaten:
**CH FR GB IT LI NL**

(56) Entgegenhaltungen:
**DE - B - 2 351 247**
**DE - B - 2 635 635**
**DE - B - 2 921 444**

(73) Patentinhaber: **DORNIER SYSTEM GmbH,
Postfach 1360, D-7990 Friedrichshafen (DE)**

(72) Erfinder: **Müller, Hans Gerhard, Dr., Sommerweg 16,
D-7990 Friedrichshafen 24 (DE)**
Erfinder: **Wess, Othmar, Dr., Hersbergweg 6,
D-7997 Immenstaad (DE)**

(74) Vertreter: **Landsmann, Ralf, Dipl.-Ing., Kleeweg 3,
D-7990 Friedrichshafen 1 (DE)**

## Beschreibung

Funkenstrecke zur Erzeugung von Stosswellen für die berührungsfreie Zertrümmerung von Konkrementen in Körpern von Lebewesen.

Die Erfindung betrifft eine Funkenstrecke zur Erzeugung von Stosswellen für die berührungsfreie Zertrümmerung von Konkrementen in Körpern von Lebewesen.

Aus der DE-PS 2 351 247 ist eine Einrichtung zum Zertrümmern von im Körper eines Lebewesens befindlichen Konkrementen mit einer Fokussierungskammer bekannt, wobei die Fokussierungskammer ein Teil eines Rotationsellipsoids ist und in deren einem Brennpunkt Stosswellen durch Funkenentladung erzeugbar sind. Wird die elektrische Unterwasserfunkenentladung in dem Brennpunkt der rotationselliptischen Fokussierungskammer gezündet, so lassen sich nahezu punktförmig im zweiten Brennpunkt Stosswellen hoher Amplitude ( >1 kbar) mit geringen Impulslängen ( <1 μsec) erzeugen. Die im Körper von Lebewesen befindlichen Konkremente können mit diesen Stosswellen in abgangsfähige Bruchstücke zertrümmert werden.

Bei der bekannten Einrichtung liegen sich zwei Elektroden gegenüber, wobei eine getrennte Zuleitung erfolgt. Zur Erzeugung von Stosswellen werden Spannungen zwischen 15 kV und 30 kV benötigt. Die Zerstörung von Konkrementen ist wesentlich von der Flankensteilheit und der Impulslänge abhängig. An die Unterwasserfunkenstrecke werden deshalb hinsichtlich der mechanischen Festigkeit und der elektrischen Eigenschaften hohe Anforderungen gestellt.

Aus der DE-PS 2 635 635 ist eine Funkenstrecke zur Erzeugung von Stosswellen für die berührungsfreie Zerstörung von Konkrementen in Körpern von Lebewesen bekannt, bei der Zuleitung und Rückleitung induktionsarm geführt sind, mit aus einer Halterung herausragenden Elektroden, wobei eine Elektrode verlängert und über eine Schleife zurückgeführt ist, so dass sich die Elektroden axial gegenüberliegen. Die Funkenstrecke ist auswechselbar und seitlich im Reflektor angebracht. Die Elektroden liegen nicht koaxial auf der grossen Halbachse des Reflektors, die durch den Reflektor gegebene Rotationssymmetrie ist durchbrochen.

Das behindert die Wellenausbreitung und kann Asymmetrien im Funkensprung verursachen. Dies gilt auch für eine Ausführungsform mit einem als Käfig ausgebildeten Aussenleiter, an dem eine zweite Elektrode befestigt ist.

Aus der DE-OS 2 418 631 ist eine Vorrichtung zum Erzeugen von Druckwellen bekannt, bei der eine Funkenstrecke im Hauptscheitel eines Reflektors angebracht ist. Ihre Elektroden liegen jedoch nicht koaxial auf der grossen Halbachse.

Der Erfindung liegt die Aufgabe zugrunde, eine Funkenstrecke zur Erzeugung von Stosswellen für die Zerstörung von Konkrementen in Körpern von Lebewesen zu schaffen mit extrem kurzer Impulslänge, die eine hohe mechanische Festigkeit aufweist, die elektrische Energie mit höherem Wirkungsgrad in fokussierte Stosswellen umwandelt, bei der Querschläger vermieden werden, wobei die Funkenstrecke so zu gestalten ist, dass eine möglichst grosse wirksame Abstrahlfläche vorhanden ist, die die Fokussierungskammer möglichst gleichmässig «ausleuchtet».

Diese Aufgabe wird erfindungsgemäss gelöst von einer Funkenstrecke gemäss dem Kennzeichen des Anspruchs 1. Ausführung der Erfindung sind Gegenstände von Unteransprüchen.

Durch die Lage der Elektroden auf der grossen Halbachse wird ein höherer Wirkungsgrad erreicht, da jetzt in der Hauptabstrahlrichtung der Stosswelle (senkrecht zu den Elektroden) eine gleichmässigere Reflexion möglich ist. Die Bügel, die bogenförmig um den Brennpunkt herumführen, verhindern Querschläger. Ihre flache Ausführung in radialer Ausrichtung gewährt eine hohe Stabilität, verbunden mit einer geringen Induktivität und minimiert die Störung der Stosswellenausbreitung. Der Funkensprung findet stets (auch bei einem gewissen Abbrand) im Zentrum des Reflektors statt. Der der Krümmung des Reflektors angepasste Schaftabschluss vergrössert die wirksame Reflektorfläche.

In vorteilhaften Ausführungsformen der Erfindung werden als Elektroden dünne Stäbchen verwendet, die zum Schutz vor Überschlägen zum Bügel hin lackiert sein können, die schnell auswechselbar und/oder nachschiebbar sein können. Die dünnen Stäbchen erfordern kein Anspitzen der Elektroden; es können billige Rundstäbe verwendet werden. Die geringen Abmessungen der Stäbchen behindern die Stosswellenabstrahlung kaum. Mit einem einfachen Nachführmechanismus kann der Abbrand ausgeglichen werden, die Standzeit erhöht sich. Verbrauchte Stäbchen können schnell ausgewechselt werden. Eine Lackschicht auf den Mantelflächen verhindert Überschläge, die nicht von den Spitzen ausgehen.

Die Erfindung wird anhand von drei Figuren näher erläutert.

Es zeigt

Fig. 1 die prinzipielle Darstellung einer Einrichtung zum Zertrümmern von im Körper eines Lebewesens befindlichen Konkrementen mit einer Unterwasserfunkenstrecke

Fig. 2 zeigt eine Ausführungsform einer Funkenstrecke in Draufsicht

Fig. 3 zeigt eine weitere Ausführungform einer Funkenstrecke mit nachführbaren, stäbchenförmigen Elektroden.

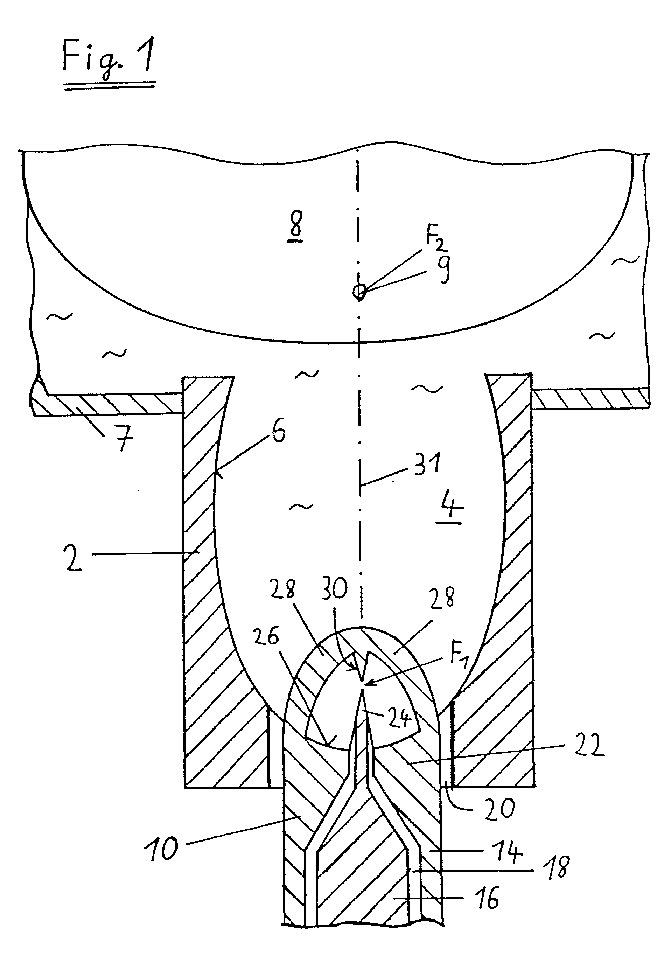

Figur 1 zeigt eine Fokussierungskammer 2, deren mit Flüssigkeit 4 gefüllter Innenraum eine in Schnittansicht ellipsenförmig verlaufende Wandung 6 aufweist. Oberhalb des Ellipsoids 2 befindet sich in einer flüssigkeitsgefüllten Wanne 7 ein Körper 8 mit einem zu zerstörenden Konkrement 9. Das Ellipsoid 2 besitzt zwei Brennpunkte F1 und F2. Im Brennpunkt F2 befindet sich das zu zerstörende Konkrement 9, z.B. ein Nierenstein. Im Brennpunkt F1 liegt die Funkenstrecke 10.

In dieser Ausführung besteht die Funkenstrecke 10 aus dem koaxial geführten Aussenleiter 14 und dem koaxial geführten Innenleiter 16, die durch die Isolation 18 voneinander getrennt sind. Die Funkenstrecke 10 ist durch die Isolation 20 nach aussen isoliert und gedämpft. Der Innenleiter 16 verjüngt sich im Schaftteil 22 und reicht als Elektrodenspitze 24 an den Brennpunkt F1 heran. Der rohrförmige Aussenleiter 14 ist im Schaftteil 22 verstärkt ausgebildet und bildet am Schaftabschluss 26 einen Teil der Reflektorfläche. Ausserhalb des Schaftabschlusses 26 ist der Aussenleiter 14 als Käfig aus den zwei Bügeln 28 ausgebildet, die bogenförmig um den Brennpunkt F1 herumführen, sich jenseits des Brennpunktes F1 treffen und die zweite Elektrodenspitze 30 tragen, die ebenfalls auf der grossen Halbachse 31 des Ellipsoids 2 liegt und nach innen weist.

Wird nun eine ausreichend hohe Spannung an die Leiter 14, 16 gelegt, so springt zwischen den Elektroden 24, 30 ein Funke über. Dieser Unterwasserfunke erzeugt in der Flüssigkeit 4 Stosswellen, die sich zur Wandung 6 hin ausbreiten, dort reflektiert werden und auf den Brennpunkt F2 fokusiert werden. Dort sind sie in der Lage, das Konkrement 9 in abgangsfähige Bruchstücke zu zertrümmern.

Der koaxiale Funkenstreckenaufbau mit zwei oder mehr Korbbügeln 28 berücksichtigt die Forderung nach niedriger Induktivität. Im Gegensatz zum Stand der Technik kann der Korbdurchmesser zum Elektrodenschaft 22 hin deutlich verbreitert werden. Bei den derzeit verwendeten Ellipsoiden (Halbachsen a = 125 mm, b = 75 mm) kann der Schaftdurchmesser z.B. 6 cm betragen, so dass die Korbbügel 28 bei den derzeit verwendeten Spannungen (bis 30 kV) mit Sicherheit so weit von der Innenelektrode 24 entfernt zum Schaft 22 geführt werden können, dass Querschläger auch ohne Bügelisolierung verhindert werden. Der schaftnahe Korbdurchmesser wird jedoch aus Gründen der Reduktion der Induktivität nicht grösser als notwendig ausgeführt. Andererseits ergibt sich eine verringerte Induktivität der Elektrodenzuführung aus einer Querschnittsvergrösserung der koaxialen Zuleitung 14, 16.

Der Abschluss 26 des Elektrodenschaftes 22 besteht in dieser Ausführung aus einem Material, das eine gute Stosswellenreflexion bei annähernd senkrechtem Einfall gewährleistet (z.B. Messing). Der Elektrodenschaft 22 passt sich nahtlos in die Reflektoroberfläche 6 ein, er wird selbst zu einem Teil des Reflektors und vergrössert so die wirksame Reflexionsleistung.

Ausführungen, bei denen der Aussenleiter 14 leitend mit dem Ellipsoid 2 verbunden ist, bei denen die Schaftreflektorfläche 26 elektrisch vom Aussenleiter 14 isoliert ist oder aus einem Isolator besteht, sind möglich, hier aber nicht gezeigt.

Die Ausführung der Korbbügel aus Flachmaterial ist gegenüber den bekannten Rundstäben besonders vorteilhaft. Die flache Ausführung in radialer Ausrichtung bietet einerseits ausreichend Querschnitt für die geforderte Stabilität und andererseits eine minimale Störung der Stosswellenausbreitung, sowohl für die primären als auch für die reflektierenden Stosswellen.

Ähnlich wie bei einem elektrischen Dipol variiert die durch Funkensprung zwischen den zwei axial angeordneten Elektroden 24, 30 abgestrahlte Stosswelle mit einem Azimutwinkel. Die grösste Amplitude wird senkrecht zur Elektrodenachse (grosse Halbachse) abgestrahlt. Bei erfindungsgemässem Elektrodeneinbau wird gerade der Teil der Primärwelle mit der höchsten Amplitude in Richtung auf die wichtigsten Reflektorzonen abgestrahlt. Unfokussiert geht nur ein ohnehin schwächerer Teil der primären Stosswelle verloren. Bei dem seitlichen Einbau der Funkenstrecke, wie er vom Stand der Technik her bekannt ist, ging ein grösserer Teil der Primärwelle mit hoher Amplitude unfokussiert zur Öffnung hinaus verloren. Das Gleiche gilt für die der Ellipsoidöffnung entgegengesetzte Abstrahlrichtung zum Ellipsoidhauptscheitel hin.

Durch die Lage der Elektroden 24, 30 auf der grossen Halbachse 31 wird auch die Symmetrie und Reproduzierbarkeit des Funkensprungs gegenüber dem Stand der Technik, bei dem die axiale Symmetrie gebrochen war, verbessert.

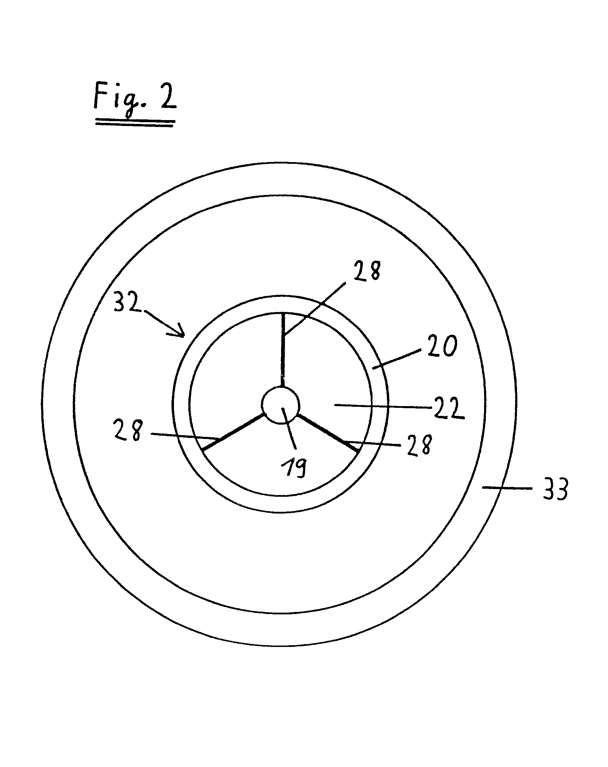

Figur 2 zeigt eine Ausführung einer Funkenstrecke 32 mit drei Bügeln 28 von der Ellipsoidöffnung aus gesehen. Innerhalb des Ellipsoids 33 sind zu erkennen: die äussere Isolation 20, der Schaftteil 22, die drei Bügel 28 und die Halterung 19 für die obere Elektrode. Verdeckt sind die innere Isolation und die beiden koaxialen Elektroden. Die schmale Ausführung der Bügel 28 stört die Ausbreitung des Stosswellenfeldes kaum.

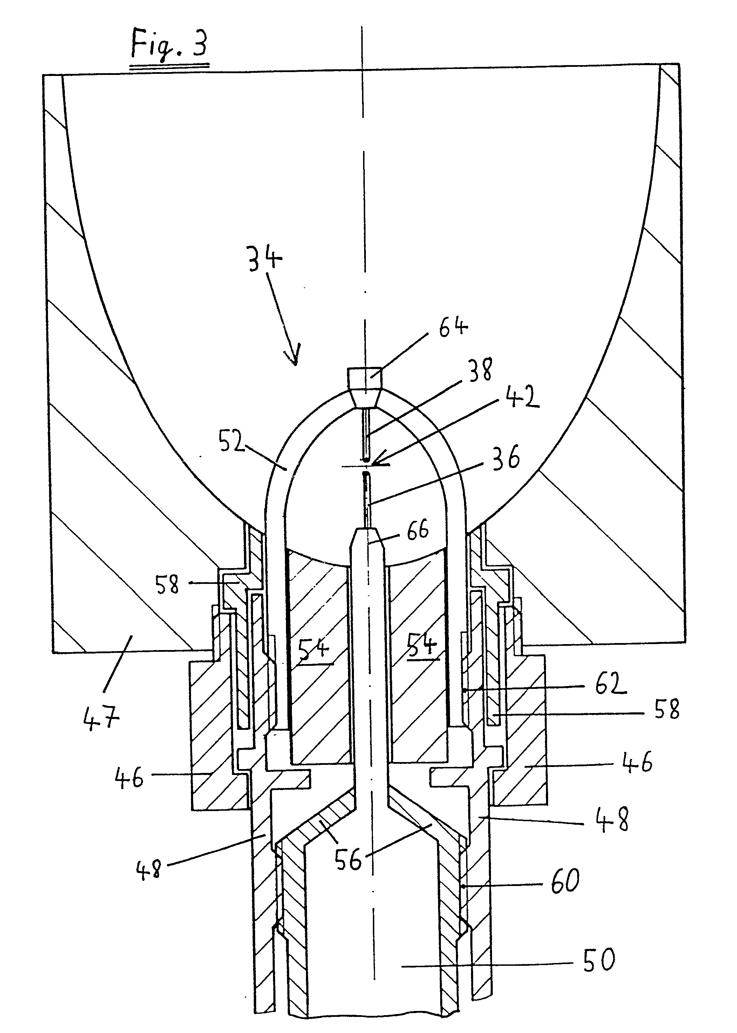

Figur 3 zeigt schematisch eine Ausführung einer Funkenstrecke 34 mit Nachstell- und Schnellwechseleinrichtung für stäbchenförmige Elektroden 36, 38.

Nachstellvorrichtungen, mit denen der Abbrand von Elektroden ausgeglichen werden kann, sind an sich bekannt. Ebenso sind Spannfutter für den schnellen Wechsel von stäbchenförmigen Körpern bekannt, z.B. Bohrfutter bei Drillbohrern.

Es wird hier eine Ausführung einer Funkenstrecke 34 gezeigt, die beim Verdrehen an einer Hülse 48 die Elektroden 36, 38 koaxial gegeneinander bewegt, so dass die eigentliche Funkenstrecke zwischen den Elektrodenspitzen immer im ersten Brennpunkt 42 des Ellipsoids bleibt.

Diese Ausführung der Funkenstrecke 34 besteht aus einer Wechselhülse 46, mit der die gesamte Funkenstrecke 34 im Reflektorkörper 47 befestigt wird. Innerhalb der Wechselhülse 46 stecken ineinander: Aussenleiter 48, Innenleiter 50 und Elektrodenkorb 52, getrennt durch die Isolationshülsen 54, 56, 58. Der Aussenleiter 48 ist als drehbare Hülse ausgestaltet und besitzt ein Innenlinksgewinde 60, mit dem der Innenleiter 50 mit Elektrode 36 nach oben (zum Brennpunkt 42 hin) verschoben werden kann, und ein Innenrechtsgewinde 62, das bei der gleichen Drehung den Elektrodenkorb 52 mit Elektrode 38 nach unten (ebenfalls zum Brennpunkt 42 hin) zieht.

Der Spitzenabstand lässt sich dadurch von aussen während einer Applikation problemlos immer wieder auf den optimalen Abstand nachjustieren. Damit lassen sich die Optimalbedingungen hinsichtlich Fokussierung, Wirkungsgrad und Kavitationsblasen konstant einhalten. Die Nachführung der Elektroden 36, 38 kann automatisiert werden. Ein schneller Austausch von abgebrannten Elektroden 36, 38 wird dadurch möglich, dass die stäbchenförmigen Elektroden 36, 38 in einer Wechselhalterung 64, 66 nach Art eines einfachen Bohrfutters mit geteilten Backen und konischer Überwurfmutter gehalten werden.

### Patentansprüche

1. Funkenstrecke zur Erzeugung von Stosswellen in einem Brennpunkt eines ellipsoidförmigen Reflektors für die berührungsfreie Zertrümmerung von Konkrementen in Körpern von Lebewesen, mit koaxial geführtem Aussenleiter und Innenleiter, wobei sich der Innenleiter zur ersten Elektrode verjüngt und der Aussenleiter in einen Käfig übergeht, an dem die zweite Elektrode befestigt ist, dadurch gekennzeichnet, dass die beiden Elektroden (24, 30) auf der grossen Halbachse (31) des Ellipsoids (2) liegen, dass der Käfig von radial ausgerichteten, bogenförmig um den Brennpunkt (F1) führenden Bügeln (28) mit flachem Querschnitt gebildet wird und dass der Aussenleiter (14) am Übergang zum Käfig als ellipsoidförmige Reflektorfläche (26) ausgebildet ist.

2. Funkenstrecke nach Anspruch 1, dadurch gekennzeichnet, dass als Elektroden dünne zylindrische Stäbchen verwendet werden, die zum Schutz vor Querschlägern mit Lack isoliert sein können.

3. Funkenstrecke nach den Ansprüchen 1 oder 2, dadurch gekennzeichnet, dass die Elektrodenstäbchen (36, 38) in Schnellspannfuttern (64, 66) leicht auswechselbar befestigt sind.

4. Funkenstrecke nach einem der vorhergehenden Ansprüche, dadurch gekennzeichnet, dass die Elektroden (36, 38) durch Verdrehen einer Hülse (48) gegeneinander verschiebbar sind.

5. Funkenstrecke nach einem der vorhergehenden Ansprüche, dadurch gekennzeichnet, dass der Käfigdurchmesser im Bereich der kleinen Halbachse des Ellipsoids liegt.

6. Funkenstrecke nach einem der vorhergehenden Ansprüche, dadurch gekennzeichnet, dass die Reflektorfläche (26) aus einem Material besteht, das Stosswellen bei annähernd senkrechtem Einfall gut reflektiert (z.B. aus Messing).

### Claims

1. Spark gap for the generation of shock waves in a focal point of an allipsoidal reflector for the non contact disintegration of concrements in living bodies, including a pair of coaxial conductors, the inner conductor changing over into a first electrode, the outer conductor forming a cage, to which the second electrode is fastened, characterised in that both electrodes (24, 30) extend along the long axis (31) of the ellipsoid (2), that the cage is formed by radial alinged, flat arches (28) and that the outer conductor (14) is formed in a contour, which merges with the ellipsoidal surface (26) of the reflector.

2. Spark gap as in claim 1, wherein the electrodes are thin cylindrical rods, coated with isolating varnish.

3. Spark gap as in claims 1 or 2, wherein the electrode rods (36, 38) are mounted in preset collars (64, 66).

4. Spark gap as in one of the preceding claims, wherein the electrodes (36, 38) are adjustable relative to each other by turning a sleeve (48).

5. Spark gap as in one of the preceding claims, wherein the diameter of the cage is approximate als long as the short axis of the ellipsoid.

6. Spark gap as in one of the preceding claims, wherein the surface (26) of the reflector is made from a material, reflecting shock waves (i.e. brass).

### Revendications

1. Eclateur pour la génération d'ondes de choc dans l'un des foyers d'un réflecteur ellipsoïdal pour la fragmentation sans contact de concrétions contenues dans les corps d'organismes vivants, avec des conducteurs extérieur et intérieur disposés coaxialement, le conducteur intérieur se rétrécissant en une première électrode et le conducteur extérieur se transformant en une cage à laquelle est fixée la deuxième électrode, caractérisé en ce que les deux électrodes (24, 30) se situent sur le grand demi-axe (31) de l'ellipsoïde (2); que la cage est constituée par des étriers (28) à section plate orientés radialement et passant en arc autour du foyer (F1); et que le conducteur extérieur (14) est conformé, dans la zone de transition vers la cage, en surface de réflecteur ellipsoïdale (26).

2. Eclateur selon la revendication 1, caractérisé en ce que de minces baguettes cylindriques sont utilisées comme électrodes et peuvent être isolées avec du vernis pour assurer la protection contre des ricochets.

3. Eclateur selon l'une des revendications 1 ou 2, caractérisé en ce que les baguettes d'électrodes (36, 38) sont fixées de manière facilement interchangeable dans des mandrins à serrage rapide (64, 66).

4. Eclateur selon l'une quelconque des revendications 1 à 3, caractérisé en ce que les électrodes (36, 38) peuvent être déplacées l'une par rapport à l'autre en tournant une douille (48).

5. Eclateur selon l'une quelconque des revendications 1 à 4, caractérisé en ce que le diamètre de la cage se situe dans la plage du petit demi-axe de l'ellipsoïde.

6. Eclateur selon l'une quelconque des revendications 1 à 5, caractérisé en ce que la surface réflectrice (26) est réalisée en un matériau qui assure une bonne réflexion des ondes de choc sous une incidence à peu près perpendiculaire (laiton, par exemple).

# Fig. 1

# Fig. 2

Fig. 3